# EUROPÄISCHE PATENTSCHRIFT

(11) **EP 0 397 876 B1**
(45) Veröffentlichungstag und Bekanntmachung des Hinweises auf die Patenterteilung: **20.09.1995**
(21) Anmeldenummer: 89902700.7
(22) Anmeldetag: 22.11.1988
(51) Int. Cl.: B21C 3/08, B21C 37/15, B21C 1/22

(54) **VERFAHREN UND VORRICHTUNG ZUR HERSTELLUNG VON PROFILROHREN FÜR BRUNNENBAUWERKE**
METHOD AND DEVICE FOR MAKING PROFILED PIPES USED FOR WELL CONSTRUCTION
PROCEDE ET DISPOSITIF POUR FABRIQUER DES TUBES PROFILES UTILISES POUR LA CONSTRUCTION DE PUITS

(43) Veröffentlichungstag der Anmeldung: 22.11.1990
(73) Patentinhaber: TATARSKY GOSUDARSTVENNY NAUCHNO-ISSLEDOVATELSKY I PROEKTNY INSTITUT NEFTYANOI PROMYSHLENNOSTI, Bugulma, 423200 (SU)
(72) Erfinder: ABDRAKHMANOV, Gabdrashit Sultanovich, Bugulma, 423200 (SU); ZAINULLIN, Albert Gabidullovich, Bugulma, 423200 (SU); BULGAKOV, Rishit Timergaleevich, Moscow, 117393 (SU); PEROV, Anatoly Vasilievich, Moscow, 113405 (SU); VAKULA, Yaroslav Vasilievich, Almetievsk, 423400 (SU); FOTOV, Alexandr Andreevich, Moscow, 127018 (SU); DUEV, Veniamin Nikolaevich, Pervouralsk, 623100 (SU); MOISEEV, Gennady Petrovich, Pervouralsk, 623100 (SU); LYASHENKO, Ivan Andreevich, Pervouralsk, 623100 (SU); SHAYAKHMETOV, Shamil Kashfullinovich, Bugulma, 423200 (SU); IBATULLIN, Rustam Khamitovich, Bugulma, 423200 (SU); ALESHIN, Vladimir Arkadievich, Pervouralsk, 623100 (SU); FROLOV, Alexandr Yakovlevich, Pervouralsk, 623100 (SU); MINGAZOV, Ilmas Falikhovich, Bugulma, 423200 (SU); VAFIN, Ildus Zakievich, rabochy poselok Shugurovo, 423282 (SU)
(74) Vertreter: Füchsle, Klaus, Dipl.-Ing.
(86) Internationale Anmeldenummer: SU8800239
(87) Internationale Veröffentlichungsnummer: WO9005598

(56) Entgegenhaltungen:
- SU-A- 0 010 823
- SU-A- 0 425 689
- SU-A- 0 827 208
- SU-A- 0 997 892
- US-A- 3 487 673

## Beschreibung

### Gebiet der Technik

Die vorliegende Erfingung bezieht sich auf das Druckumformen und betrifft ein Verfahren und eine Vorrichtung zur Herstellung von Profilrohren für Brunnenbauwerke, die beim Anlegen von Bohrungen verwendet werden.

Am wirkungsvollsten kann die vorliegende Erfindung bei der Herstellung von Profilrohren, die zum Schließen erschwerter Zonen beim Niederbringen von Bohrungen verwendet werden, eingesetzt werden.

### Zugrundeliegender Stand der Technik

Beim Niederbringen von Tiefbohrungen kommt es häufig vor, daß die angebohrten Gesteinsschichten intensiv die Bohrspülung aufnehmen oder in die Bohrung Schichtwasser absondern. Die Isolierung solcher Schichten mit gewöhnlichen Methoden durch Zementieren führt nicht zum gewünschten Ergebnis. Heutzutage werden in solchen Fällen metallische Kassettenabdeckungen, vorher aufgewickelte, volle (vom Bohrlochmund) Zwischenrohrstränge oder verkürzte Rohrstränge eingebracht.

Die Abdeckungen haben jedoch keine weite Verbreitung gefunden, da sie keinen dichten Abschluß der erschwerten Zonen gewährleisten. Außerdem ist ihre Länge begrenzt, weshalb mit ihrer Hilfe erschwerte Zonen, die zehn und hunderte Meter messen effektiv nicht isoliert werden können.

Die Anwendung von Zwischen- oder verkürzten Rohrsträngen gewährleistet ein sicheres Schließen der erschwerten Zonen. Diese Lösung ist jedoch sehr materialaufwendig, die erwähnten Rohrstränge müssen in der Bohrung zementiert werden, große Mengen an Stahl, Zement und Zeit sind erforderlich. Außerdem verringert sich der Durchmesser der Bohrung beim Einbringen jedes zusätzlichen Rohrstrangs, wodurch der Betrieb der Bohrung erschwert wird.

### Kennzeichnung einer bekannten technischen Lösung

Es ist ein Verfahren zur Herstellung von Profilrohren bekannt, das das Profilieren des Mittelteils eines zylindrischen Rohres beinhaltet, bei dem das Rohr durch ein formbildendes Element gezogen wird (SU, A, 549196).

Die Vorrichtung zu dessen Realisierung enthalt einen Ziehstein, der aus einer Hülse mit einer Profilmatrize in Form geteilter Elemente besteht, die auf elastischen Stäben angebracht sind, die durch einen Ring verbunden sind, und eine Einrichtung zur Erzeugung einer äußeren Lasteinwirkung auf die Profilmatrize. Die elastischen Stäbe sind in einer Entfernung vom Stirnende der Matrize miteinander verbunden, die nicht weniger als zwei Langen der Elemente der Matrize beträgt.

Der Hauptnachteil des bekannten Verfahrens und der Vorrichtung zu seiner Realisierung besteht darin, daß die auf diese Weise hergestellten Profilrohre in das Bohrloch nicht eingebracht und in der erschwerten Zone mit dichtem Andrücken an die Bohrlochwände nicht installiert werden können, da das Ausgangsrohr vor dem Profilieren einen Außendurchmesser haben muß, der dem Durchmesser das Bohrlochs in der erschwerten Zone gleicht.

Beim Profilieren von Rohren nach dem bekannten Verfahren verringert sich der Durchmesser des Rohrs jedoch nur im mittleren, profilierten Teil des Rohrs. Die zylindrischen Enden der Rohre behalten ihren ursprünglichen Durchmesser und passen nätürlich in das Bohrloch nicht hinein. Wenn man den Durchmesser des Rohrs verringert, kann das Rohr in der erschwerten Zone nicht installiert werden, da die Rohrwand an die Bohrlochwand nicht angedrückt wird. Dieser Nachteil tritt noch stärker in Erscheinung, wenn beim Schließen der erschwerten Zone ihr Durchmesser relativ zum Bohrlochdurchmesser erweitert wird, damit der Durchgangskanal des Bohrlochs nicht verkleinert werden muß.

Ein weiterer Nachteil des bekannten Verfahrens und der Vorrichtung zu seiner Realisierung besteht darin, daß der Herstellungsprozeß eines Profilrohrs mit zwei zylindrischen Enden mehrere technologische Arbeitsgänge umfaßt, wodurch der Herstellungsprozeß erschwert und verteuert wird und die Arbeitsproduktivität sinkt.

Es ist ein Verfahren zur Herstellung von Profilrohren bekannt, bei dem die Rohre durch ein formbildendes Werkzeug gezogen werden (Shurupov A.K., Freiberg M.A. "Die Produktion von Rohren mit Sparprofilen", 1963, Staatlicher wissenschaftlich-technischer Verlag der Schwarz- und Buntmetallurgie, Sverdlovsk, S. 146). Das vorgegebene Profil des Rohrs wird unverändert auf dessen gesamter Länge ausgeführt.

Der Nachteil dieses Verfahrens besteht darin, daß die nach diesem Verfahren hergestellten Rohre zu einem Rohrstrang durch Verschweißen ihrer Enden miteinander verbunden werden, was unter nichtstationären Bedingungen am Bohrloch äußerst schwierig ist. Außerdem werden zum Einbringen und Aufstellen der Rohre im Bohrloch komplizierte Vorrichtung benötigt, und zwar Spann- und Preßköpfe.

Schließlich ist aus der SU-A-82 72 08 eine Vorrichtung bekannt, die eine Profilmatrize aufweist, die aus mehreren, in einem Haltekörper befestigten Teilen zusammengesetzt ist. Um die Herstellung von profilierten Rohren mit einem unprofilierten glatten Ende zu ermöglichen, wobei das glatte unprofilierte Ende den gleichen Umkreis-Durchmesser aufweist wie der Profilteil, ist zusätzlich ein einstückiger Ziehstein vorgesehen, mit glatten Arbeitsflächen, der in Arbeitsrichtung gesehen vor der Profilmatrize angeordnet ist. Der Rohrrohling wird zuerst durch den Ziehstein hindurchgeführt, der den Rohrdurchmesser reduziert. Anschließend wird das Rohr mit reduziertem Durchmesser durch die Profilmatrize hindurchgeführt, die das entsprechende Profil einarbeitet. Nach Erreichen einer bestimmten Länge des profilierten Rohrteils wird das Ziehen unterbrochen und die Profilier-Matrize wird inaktiviert, d. h., die Profilierteile werden zurückgezogen, so daß beim nachfolgenden weiteren Ziehen das verbleibende Ende des Rohres nicht mehr profiliert, sondern nur noch kalibriert wird. Durch diese bekannte Vorrichtung wird somit ein Profilrohr hergestellt, das zwar durchgehend einen gleichen, reduzierten Durchmesser besitzt, jedoch nur ein nicht profiliertes glattes Rohrende aufweist. Die hierdurch erhaltenen Profilrohre sind nicht geeignet, miteinander durch Schweißen oder Schraubverbindungen zu einem entsprechenden Rohrstrang zusammengesetzt zu werden.

Die vorliegende Erfindung bezweckt die Herstellung von Profilrohren mit zylindrischen Enden, die zum Schließen erschwerter Zonen in Bohrlöchern ohne Verringerung des Durchgangsdurchmessers der Bohrungen verwendet werden können.

Ein weiteres Ziel der vorliegenden Erfindung besteht in der Vereinfachung und Verbilligung des technologischen Herstellungsprozesses von Profilrohren.

Des weiteren verfolgt die vorliegende Erfindung das Ziel, die Arbeitsproduktivität zu erhöhen.

Der vorliegenden Erfindung liegt die Aufgabe zugrunde, ein Verfahren zur Herstellung von Profilrohren für Brunnenbauwerke, die beim Niederbringen von Bohrungen verwendet werden, und eine Vorrichtung zu dessen Realisierung zu schaffen, die die Herstellung von Profilrohren mit zwei äußeren zylindrischen Teilen gewährleisten, deren Durchmesser im wesentlichen dem Durchmesser des Umkreises ihres Profilteils gleicht.

### Offenbarung der Erfindung

Die gestellte Aufgabe wird durch ein Verfahren mit den Merkmalen des Auspruchs 1 gelöst.

Das erfindungsgemäße Verfahren ermöglicht durch die Ausführung des Profilteils und der zylindrischen End-Teile des Rohrs mit gleichen Außenabmessungen des Querschnitts, den aus Profilrohren bestehenden Rohrstrang frei in die erschwerte Zone des Bohrlochs hinabzulassen und nach dem Ausweiten der Profilrohre zuverlässig diese Zone zu schließen, indem die Rohre fest an die Bohrlochwand angedrückt werden.

Die gestellte Aufgabe wird auch durch eine Vorrichtung mit den Merkmalen des Anspruchs 2 gelöst.

Solch eine Ausführung der Konstruktion der Vorrichtung ermöglicht es, durch Verkürzung der technologischen Arbeitsgange zur Weiterbewegung des Rohrs mit dem Zweck, von ihm das formbildende Element nach dem Profilieren des Mittelteils des Rohrs wegzuführen, den Herstellungsprozeß von Profilrohren mit zylindrischen Enden zu vereinfachen, zu beschleunigen und zu verbilligen und diesen Vorgang ununterbrochen zu gestalten, die technologischen Arbeitsgänge zu automatisieren, die Arbeit des Bedienungspersonals zu erleichtern und folglich die Arbeitsproduktivität zu erhöhen.

In einer zu bevorzugenden Variante der Erfindung sitzt auf einer jeden der Nocken (6) verschwenkbar und koaxial zu deren Schwenk-Achse (4) eine Scheibe (16) auf, und einer jeden Scheibe (16) ist ein zweigliedriger Hebel (21) zugeordnet, dessen ein Glied (23) am Gehäuse (1) und dessen anderes Glied (22) an der Scheibe (16) angelenkt ist, wobei die Scheiben (16) und die Nocken (6) für eine gegenseitige formschlüssige Schwenk-Mitnahme ausgebildet sind, und die Stützen (26) über die zweigliedrigen Hebel (21) arretierend auf die Nocken (6) einwirken.

Das ermöglicht es, die Belastung der Stützen zu verringern und dadurch ihre Lebensdauer zu verlangern.

### Kurze Beschreibung der Zeichnungen

Weitere Ziele und Vorteile der vorliegenden Erfindung werden aus folgender ausführlicher Beschreibung von Ausführungsbeispielen und den beiliegenden Zeichnungen verständlich, und zwar zeigt:
Fig. 1 die Gesamtansicht einer Vorrichtung gemäß der Erfindung;
Fig. 2 die erfindungsgemäße Vorrichtung in der Draufsicht;
Fig. 3 eine Nocke (Draufsicht);
Fig. 4 eine Nocke (Seitenansicht);
Fig. 5 eine Scheibe (Draufsicht);
Fig. 6 eine Scheibe (Seitenansicht);
Fig. 7 den Getriebeplan von zweigliedrigen Mechanismen mit Scheiben und Nocken in der Ausgangsstellung vor dem Profilieren des Rohrs;
Fig. 8 wie Fig. 7, in der Arbeitsstellung;
Fig. 9 wie Fig. 7, zum Zeitpunkt der Beendigung des Profilierens des Rohrs;
Fig. 10 das Schema der gegenseitigen Anordnung der Arme eines zweigliedrigen Gelenkmechanismus.

### Beste Ausführungsvariante der Erfindung

Das Verfahren zur Herstellung von Profilrohren besteht in folgendem.

Einen zylindrischen Rohrrohling zieht man durch ein formbildendes Werkzeug, wo der mittlere Teil des Rohrs profiliert wird und das Reduzieren des Rohrs auf seiner gesamten Länge vorgenommen wird, wobei die zylindrischen Enden des Rohrs im wesentlichen bis zum Durchmesser des Umkreises des profilierten Teils des Rohrs reduziert werden, und schneidet dann z.B. ein Gewinde auf ihnen zur Verbindung der profilierten Rohre miteinander.

Wenn einige Profilrohrpaare miteinander durch Schweißen verbunden werden sollen, läßt man beim Profilieran jedes dieser Rohre ein zylindrisches Ende frei. Das Reduzieren dar zylindrischen Enden der Rohrrohlinge kann sowohl vor dem Profilieren, als auch danach vorgenommen werden.

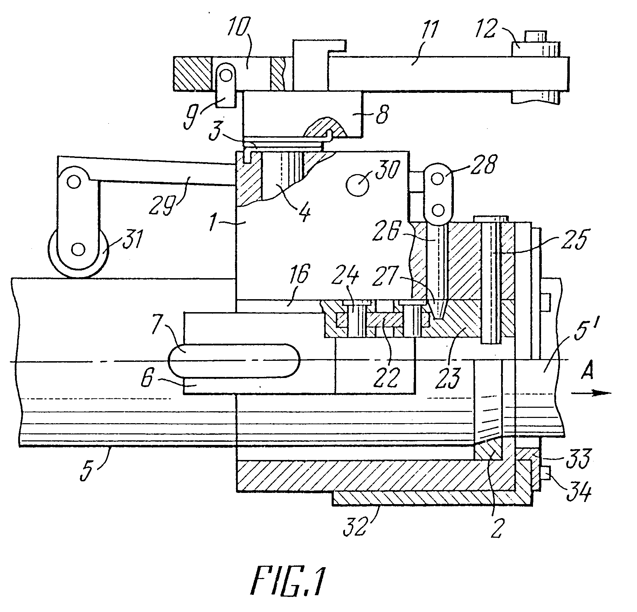

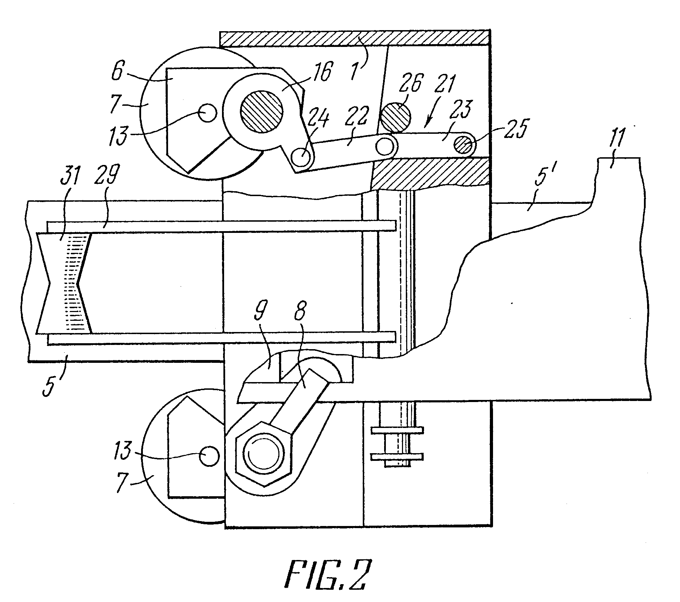

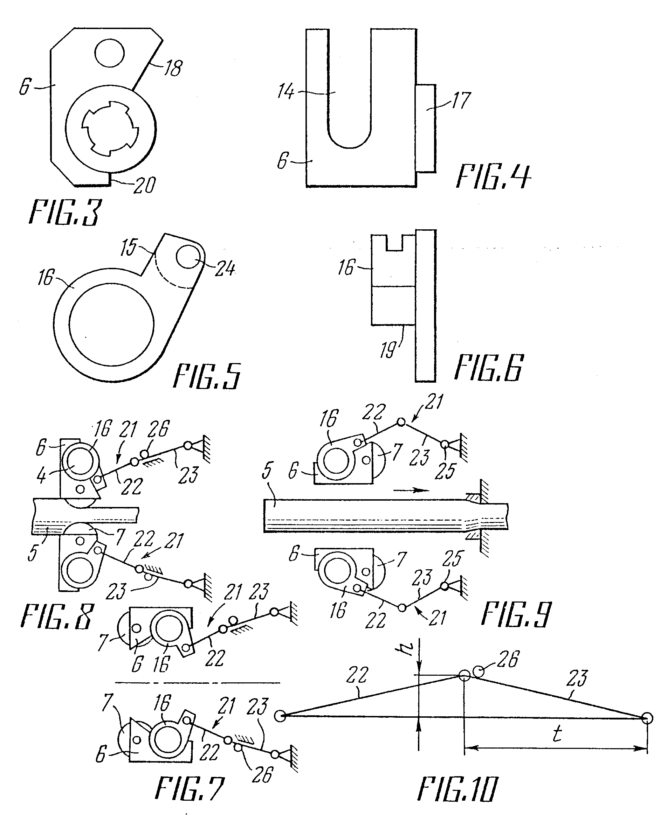

Die erfindungsgemäße Vorrichtung zur Realisierung dieses Verfahrens enthält ein Gehause 1 (Fig. 1) mit einem darin eingebauten Ziehstein 2 und senkrecht im Gehäuse 1 mit Hilfe einer Feder 3 federnd aufgestellte Achsen 4 mit Schlitzen an den Enden (in der Figur nicht abgebildet). Auf die unteren Enden der Achsen 4 sind auf beiden Seiten der Bewegungsbahn das zylindrischen Rohrrohlings 5 Nocken 6 mit Verformungsrollen 7 und auf die oberen Enden Gabelhebel 8 aufgesetzt. Die letzteren können mit Rasten 9 zusammanwirken, die an Gelenken in Nuten 10 einer Zugstange 11 aufgehängt sind, die an der Achse 12 eines Ziehwagens (in der Fig. nicht abgebildet) befestigt ist. Die Verformungsrollen 7 sind mit Hilfe von Achsen 13 (Fig. 2) in Nuten 14 (Fig. 4) der Nocken 6 angebracht und werden in der Arbeitsstellung durch Stützflächen 15 (Fig. 5) der herausragenden Teile von Scheiben 16, die drehbar auf zylindrischen Vorsprüngen 17 der Nocken 6 (Fig. 4) angebracht sind, durch Kontaktaufnahme mit Stützflächen 18 (Fig. 3) fixiert und in der Ruhestellung - durch Kontaktaufnähme von Stützflächen 19 der Scheiben 16 (Fig. 6) mit Stützflächen 20 der Nocken 6 (Fig. 3) festgehalten. Der Drehwinkel der Scheiben 16 wird durch zweigliedrige Hebel 21 mit Gliedern 22 und 23 (Fig. 1,2 und 8) begrenzt, die gelenkig am Gehäuse 1 und an den Scheiben 16 mit Hilfe von Achsen 24, 25 befestigt sind. Die Glieder 22 und 23 werden durch Stützen 26 festgehalten, die in Form von Stäben mit kegelförmiger Oberfläche 27 (Fig. 1) am unteren Ende ausgeführt und senkrecht im Gehäuse 1 hin- und herbeweglich aufgestellt sind. Die Stützen 26 sind mit den oberen Enden gelenkig mit Hilfe von Laschen 28 mit einem Ende eines Drehhebels 29 verbunden, der wiederum gelenkig mit dem Gehäuse 1 mit Hilfe einer Achse 30 verbunden ist, während sein anderes Ende mit einer Stützrolle 31 ausgerüstet ist. Der Drehhebel 29 dreht sich relativ zum Gehäuse auf der Achse 30 und ist parallel zur Längsachse der Vorrichtung angebracht. Durch die Länge des Drehhebels 29 von seiten der Stützrolle 31 legt man die Länge des zylindrischen Endes des Rohrs 5 fest, mit der die Stützrolle 31 zusammenwirkt. Die Vorrichtung befestigt man vorher an die Lünette 32 einer Ziehbank (in der Zeichnung nicht abgebildet) mit Hilfe eines Anschlagrings 33 und Schrauben 34 (Fig. 1). Das Ende des Drehhebels 29 mit den Stützen 26 befindet sich in der Ausgangsstellung in angehobener Lage, während die Verformungsrollen 7 unter Einwirkung der Feder 3 zur Seite geführt sind.

### Die Vorrichtung funktioniert folgendermaßen.

In den Ziehstein 2 führt man den zu profilierenden zylindrischen Rohrrohling 5 mit vorbereitetem (eingewalztem) Ende 5^{l} zu dessen Erfassen durch den Ziehwagen ein. Dabei wird die mit dem Rohr 5 zusammenwirkende Stützrolle 31 angehoben (Fig. 1, und das andere Ende des Drehhebels 29 mit den Stützen 26 senkt sich nach unten zum darauffolgenden Anschlag der Glieder 23 an ihre kegelförmigen Flächen 27. Die Verformungsrollen 7 sind unter Einwirkung der Feder 3 (Fig.1) zur Seite geführt (Fig. 2 und 7).

Danach wird an die Vorrichtung der Ziehwagen zum Ergreifen des vorbereiteten Endes 5^{l} des Rohrs 5 herangeführt. Dabei läuft ein Teil der Zugstange 11 mit den Rasten 9 über die Hebel 8, und ragt ein bestimmtes stück hervor, das die Länge des vorderen zylindrischen Endes des zu profilierenden Rohrs 5 bestimmt. Beim Arbeitshub des Ziehwagens bewegt sich das Rohr 5 in Pfeilrichtung A entsprechend Fig. 1. Das zylindrische Ende des Rohrs 5 läuft durch den Ziehstein 2, wird reduziert und nimmt das gewünschte Maß an. Bei Beendigung des Reduzierens der vorgesehenen Länge des vorderen Endes des Rohrs stoßen die Rasten 9 der Zugstange 11 auf die Gabelhebel 8 auf. Unter der Krafteinwirkung der Rasten 9 drehen sich die Gabelhebel 8 in Ziehrichtung und drehen wiederum über die Achsen 4 die Nocken 6 mit den Verformungsrollen 7. Die letzteren werden in die Wand des Rohrs 5 soweit eingedrückt, bis die Nocken 6 mit ihren Stützflächen 18 (Fig. 3) auf die Flächen 15 der Scheiben 16 (Fig. 5) aufstoßen, wodurch die Arretierung der Verformungsrollen 7 in der Arbeitsstellung gewährleistet wird (Fig. 8), da das Drehen der Scheiben 16 dabei von den Gliedern 23 verhindert wird, deren Drehung zur Seite (relativ zum Rohrrohling) durch die Stützen 26 aufgehalten wird. Die kegelförmigen Flächen 27 der Stützen 26 (Fig. 1) nehmen eine wesentlich geringere als die durch das Profilieren entstehende Kraft auf. Bei der Drehung der auf der Achse 4 sitzenden Hebel 8 um einen Winkel, bei dem die Arbeitsstellung der Rollen 7 gewährleistet wird, wird die Kupplung zwischen den Rasten 9 der Zugstange 11 und den Hebeln 8 gelöst. Bei der weiteren Bewegung des Rohrrohlings 5 erfolgt gleichzeitig das Profilieren und das Reduzieren des mittleren Teils des Rohrs 5 mit Hilfe des Ziehsteins 2 derart, daß der Durchmesser des Profilteils des Rohrs 5 im wesentlichen gleich dem Durchmesser des reduzierten zylindrischen Endes d^{l} des Rohrs 5 ist.

Sobald die Stützrolle 31 das Ende des Rohrs 5 erreicht, senkt sie sich unter ihrem Gewicht schnell nach unten und löst die Stützen 26 aus der Verkupplung mit den Gliedern 23, die sich auf den Achsen 25 vom Rohr 5 wegdrehen (Fig. 9), während die mit den Gliedern 22 über die Scheiben 16 verbundenen Nocken 6 sich in Ziehsichtung drehen, wobei die Verformungsrollen 7 den Kontakt mit dem Rohr 5 verlieren. Das verbliebene, nichtprofilierte zweite zylindrische Ende des Rohrs 5 läuft durch den Ziehstein 2 und wird im wesentlichen bis zum Durchmesser des reduzierten zylindrischen Endes 5^{l} reduziert (Fig. 1). Die Federn 3 bringen die Nocken 6 mit den Rollen 7 in die Ausgangsstellung zurück (Fig. 7).

Damit ist das mit dem Reduzieren des Rohrs 5 gekoppelte Profilieren des Rohrs 5 beendet.

### Gewerbliche Anwendbarkeit

Die Erfindung kann bei der Herstellung von Profilrohren eingesetzt werden, die zum Schließen von erschwerten Zonen beim Niederbringen von Bohrungen und bei der Instandsetzung von Futterrohrkolonnen zum Einsatz gelangen.

## Patentansprüche

1. Verfahren zur Herstellung von Profilrohren für Brunnenbauwerke, die beim Niederbringen von Bohrungen verwendet werden, bei dem das Rohr auf einem Teil seiner Länge profiliert wird und ein glattes, nicht profiliertes Ende beibehalten wird, wobei der Durchmesser des nicht profilierten glatten Endes des Rohres in wesentlichen dem Durchmesser des Umkreises des Profilteils des Rohres gleicht, durch Durchziehen eines zylindrischen Rohrrohlings durch einen glatten Ziehstein und durch ein in aktiver Stellung befindliches profilbildendes Werkzeug, das nach Erreichen eines vorbestimmten Rest-Rohrendes deaktiviert wird, so daß das verbleibende, zylindrische Rohrende nur noch durchmesserreduziert und nicht mehr profiliert wird,
**dadurch gekennzeichnet**,
a)- daß der Rohrrohling mit seinem einen, vorderen Ende zuerst durch das außer Aktivität gesetzte, profilbildende Werkzeug und danach durch den Ziehstein geführt wird, und
b)- daß nach Erreichen der vorgesehenen Länge des nichtprofilierten vorderen Endes des Rohres das profilbildende Werkzeug aktiviert wird, wodurch gleichzeitig das Profilieren und das Durchmesserreduzieren des mittleren Teiles des Rohres erfolgt,
wodurch das Rohr nur im mittleren Rohrteil profiliert wird und beide Enden des Rohres nicht profiliert, glatt und mit gleichem Durchmesser mit dem Umkreis des Profilteils des Rohres erhalten werden.

2. Vorrichtung zur Realisierung des Verfahrens nach Anspruch 1, die, auf einer Ziehbank montiert, einen in einem Gehäuse (1) installierten Ziehstein (2) und einen Ziehwagen enthält, dadurch gekennzeichnet,
daß vor der Eintrittsöffnung des Ziehsteins (2) einander gegenübergestellte Verformungsrollen (7) an den einen Enden von zwischen einer Arbeitsstellung und einer Ruhestellung bewegbaren Nocken (6) angebracht sind, wobei zur Herstellung einer Längsprofilierung die Verformungsrollen (7) in der Arbeitsstellung der Nocken (6) sich auf der Wand des sich in Bearbeitung befindlichen Rohrs (5) abwälzen, daß die Nocken (6) mittels an ihren anderen Enden befestigten Gabelhebeln (8) in ihrer Arbeitsstellung versetzbar und arretierbar sind, wobei die Gabelhebel (8) durch Rasten (9), die in Nuten (10) einer am Ziehwagen befestigten Zugstange (11) angebracht sind, betätigbar sind, und daß einer der Arme eines am Gehäuse (1) angeordneten Drehhebels (29) mit einer eine vorbestimmte Distanz vor der Eintrittsöffnung des Ziehsteins (2) angeordneten und auf den Umfang des sich in Bearbeitung befindlichen Rohrs (5) abrollenden Stützrolle (31) versehen ist, wobei die Arretierung der Nocken (6) mittels am anderen Arm des Drehhebels (29) gelenkig befestigten Stützen (26) aufgehoben wird, wenn sich das in Bearbeitung befindliche Rohr (5) nicht mehr im Bereich der Stützrolle (31) befindet.

3. Vorrichtung nach Anspruch 2, dadurch gekennzeichnet, daß auf jeder der Nocken (6) verschwenkbar und koaxial zu deren Schwenk-Achse (4) eine Scheibe (16) angeordnet ist, daß jeder Scheibe (16) ein zweigliedriger Hebel (21) zugeordnet ist, dessen eines Glied (23) am Gehäuse (1) und dessen anderes Glied (22) an der Scheibe (16) angelenkt ist, die Scheiben (16) und die Nocken (6) für eine gegenseitige formschlüssige Schwenk-Mitnahme ausgebildet sind, und die Stützen (26) über die zweigliedrigen Hebel (21) arretierend auf die Nocken (6) einwirken.

## Claims

1. Method of producing profiled tubes for well construction, which are used in the sinking of boreholes, wherein the tube is profiled over part of its length and retains a smooth, unprofiled end and wherein the diameter of the smooth, unprofiled tube end is substantially equal to the diameter of the circumscribed circle of the profiled part of the tube, by drawing a cylindrical tube blank through a smooth drawing die and through a profiling tool which is in its active position and which is deactivated on reaching a predetermined residual tube end, so that the remaining, cylindrical tube end is then only reduced in diameter and is not profiled, characterized
a) in that the tube blank is guided, by its one, front end, first through the deactivated profiling tool and then through the drawing die, and
b) in that after reaching the prescribed length of the unprofiled front end of the tube the profiling tool is activated, whereby the profiling and the reduction of diameter of the middle part of the tube are effected simultaneously, whereby the tube is profiled only in its middle part and the two ends of the tube are obtained unprofiled, smooth and having the same diameter as the circumscribed circle of the profiled part of the tube.

2. Apparatus for applying the method according to Claim 1, which is mounted on a drawbench and contains a drawing die (2), installed in a casing (1), and a drawing carriage, characterized in that shaping rollers (7) arranged opposite one another are disposed, in front of the inlet opening of the drawing die (2), at one end of respective cams (6) which are movable between a working position and a position of rest, while in order to effect longi- tudinal profiling the shaping rollers (7) roll on the wall of the tube (5) undergoing machining in the working position of the cams (6), in that the cams (6) can be put into and locked in their working position by means of forked levers (8) fastened to their other ends, the forked levers (8) being able to be actuated by catches (9) which are arranged in slots (10) in a tie rod (11) fastened to the drawing carriage, and in that one of the arms of a rotary lever (29) arranged on the casing (1) is provided with a support roller (31) arranged at a predetermined distance in front of the inlet opening of the drawing die (2) and rolling on the circumference of the tube (5) undergoing machining, the locking of the cams (6) being released by means of supports (26) articulatedly fastened to the other arm of the rotary lever (29) when the tube (5) undergoing machining is no longer in the region of the support roller (31).

3. Apparatus according to Claim 2, characterized in that on each of the cams (6) a disc (16) is mounted swivellably and coaxially to their swivel axis (4), in that each disc (16) has associated with it a two-membered lever (21) of which one member (23) is articulated on the casing (1) and whose other member (22) is articulated on the disc (16), the discs (16) and the cams (6) are designed for mutual positive swivelling driving, and the supports (26) apply a locking action to the cams (6) via the two-membered levers (21).

## Revendications

1. Procédé de fabrication de tubes profilés pour des constructions de puits, utilisés lors du fonçage de £orages, pour lequel le tube est profilé sur une partie de sa longueur en conservant une extrémité lisse non-profilée, le diamètre de l'extrémité lisse non-profilée du tube étant sensiblement égal au diamètre du cercle de périphérie de la partie profilée du tube, par étirage d'une ébauche tubulaire cylindrique, dans une. filière lisse et au moyen d'un outil à action profilante se trouvant en position active et qui est désactivé lorsqu'il ne reste qu'une extrémité de tube résiduelle prédéterminée, de sorte que l'extrémité de tube cylindrique restante n'est plus soumise qu'à une réduction du diamètre et non plus à un profilage, caractérisé en ce que
a)- l'ébauche tubulaire est d'abord guidée, par une première extrémité avant, à travers l'outil à action de profilage mis hors d'action, puis est guidée à travers la filière, et
b)- en ce que, après atteinte de la longueur prévue de l'extrémité avant non-profilée du tube, l'outil à action de profilage est activé, de sorte que simultanément sont effectués le profilage et la réduction de diamètre de la partie médiane du tube, de sorte que le tube n'est profilé que dans la partie médiane et que les deux extrémités du tube ne sont pas profilées, sont lisses et sont dotées du même diamètre que le cercle périphérique de la partie profilée du tube.

2. Dispositif de mise en oeuvre du procédé selon la revendication 1, monté sur un banc d'étirage, contenant une filière (2) installée dans un carter (1) et un chariot d'étirage, caractérisé en ce que, devant l'ouverture d'entrée de la filière (2), sont montés des galets de déformation (2) placés à l'opposé les uns des autres et disposés à une extrémité d'ergots (6) déplaçables entre une position de travail et une position de repos, les galets de déformation (7), lorsque les ergots (6) se trouvent en position de travail, roulant sur la paroi du tube (5) se trouvant en cours d'usinage, en vue d'établir un profilage longitudinal, en ce que les ergots (6) sont susceptibles d'être décalés et bloqués dans leur position de travail, au moyen de leviers fourchus (8) fixés sur leur autre extrémité, les leviers fourchus (8) pouvant être actionnés au moyen de cliquets (9) montés dans des rainures (10) d'une barre de traction (11) fixée sur le chariot d'étirage, et en ce que l'un des bras d'un levier tournant (29) disposé sur le carter (1) est pourvu d'un galet d'appui (31), disposé à une distance prédéterminée en avant de l'ouverture d'entrée de la filière (2) et roulant sur la périphérie du tube (5) se trouvant en cours d'usinage, le blocage des ergots (6) étant supprimé au moyen d'appuis (26) fixés articulés à l'autre bras du levier tournant (29) lorsque le tube (5) se trouvant en cours d'usinage ne se trouve plus dans la zone du galet d'appui (31).

3. Dispositif selon la revendication 2, caractérisé en ce qu'un disque (16) est disposé sur chacun des ergots (6), de façon à pouvoir pivoter et à être placé coaxialement par rapport à leur axe de pivotement (4), en ce qu'à chaque disque (16) est associé un levier à deux éléments (21), dont un élément (23) est articulé sur le carter (1) et dont l'autre élément (22) est articulé sur le disque (16), les disques (16) et les ergots (6) étant réalisés pour produire un entraînement de pivotement mutuel avec ajustement de forme et les appuis (26) agissant avec un effet de blocage sur les ergots (6), par l'intermédiaire des leviers à deux éléments (21).
